# EUROPEAN PATENT SPECIFICATION

(11) **EP 2 010 666 B1**
(45) Date of publication and mention of the grant of the patent: **16.06.2010**
(21) Application number: 07732468.9
(22) Date of filing: 19.04.2007
(51) Int. Cl.: C12P 41/00, C12P 7/22, C12P 7/62

(54) **PROCESS FOR DYNAMIC KINETIC RESOLUTION (DKR) OF RACEMIC COMPOUNDS IN (HYDRO) FLUOROCARBON SOLVENTS**
VERFAHREN FÜR DIE DYNAMISCHE KINETISCHE RACEMATSPALTUNG (DKR) RACEMISCHER VERBINDUNGEN IN FLUORKOHLEN(WASSER)STOFFLÖSUNGSMITTELN
PROCÉDÉ POUR LE DÉDOUBLEMENT CINÉTIQUE DYNAMIQUE (DKR) DE COMPOSÉS RACÉMIQUES DANS DES SOLVANTS (HYDRO)FLUOROCARBONÉS

(30) Priority: 21.04.2006 GB 0607911
(43) Date of publication of application: 07.01.2009
(73) Proprietor: Ineos Fluor Holdings Limited, Runcorn, Cheshire WA7 4QF (GB)
(72) Inventor: MICKLEFIELD, Jason, Cheshire SK4 3LZ (GB); BALL, Anthony, John, Newcastle NE6 4LP (GB); CORR, Stuart, Cheshire WA4 5DH (GB)
(74) Representative: Probert, Gareth David
(86) International application number: PCT/GB2007/001426
(87) International publication number: WO 2007/129018

(56) References cited:
- WO-A-01/28970
- WO-A-03/018531
- WO-A-03/076384
- WO-A-2004/083444
- JP-A- 2 273 634
- CHOI JUN HO ET AL: "Aminocyclopentadienyl ruthenium chloride: catalytic racemization and dynamic kinetic resolution of alcohols at ambient temperature." ANGEWANDTE CHEMIE (INTERNATIONAL ED. IN ENGLISH) 2 JUL 2002, vol. 41, no. 13, 2 July 2002 (2002-07-02), pages 2373-2376, XP002445524 ISSN: 1433-7851
- CHOI JUN HO ET AL: "Aminocyclopentadienyl ruthenium complexes as racemization catalysts for dynamic kinetic resolution of secondary alcohols at ambient temperature." JOURNAL OF ORGANIC CHEMISTRY, vol. 69, no. 6, 19 March 2004 (2004-03-19), pages 1972-1977, XP002445525 ISSN: 0022-3263
- PERSSON A: "Ruthenium- and enzyme-catalyzed dynamic kinetic resolution f secondary alcohols" JOURNAL OF THE AMERICAN CHEMICAL SOCIETY, AMERICAN CHEMICAL SOCIETY, WASHINGTON, DC, US, vol. 121, no. 8, 3 March 1999 (1999-03-03), pages 1645-1650, XP002158577 ISSN: 0002-7863
- REETZ M T ET AL: "LIPASE-CATALYZED DYNAMIC KINETIC RESOLUTION OF CHIRAL AMINES: USE OF PALLADIUM AS THE RACEMIZATION CATALYST" CHIMIA, AARAU, CH, vol. 50, no. 12, 1 December 1996 (1996-12-01), pages 668-669, XP009036319 ISSN: 0009-4293

## Description

The present invention relates to a process for preparing a third compound by catalytic conversion of a first compound and a second compound More particularly, the invention relates to a process for stereo-selactively preparing a third compound by reacting a substrate comprising a first enantiomeric compound with a racemisation catalyst and also reacting the second enantiomeric compound, in the presence of an enzyme.

Catalysts are materials that act to increase the rates of reactions without themselves being consumed by the reaction. Enzymes are biological catalysts that in many cases are sufficiently effective to reduce reaction activation energies to the point where the reaction becomes diffusion limited.

An outstanding feature of enzyme catalysis is the observed substrate specificity, which determines biological function. Some enzymes utilise only one biological substrate and are said to exhibit absolute substrate specificity. For example, glucokinase will catalyse the transfer of phosphate from ATP to glucose but to no other sugar. Other enzymes display much broader substrate specificity and are able to utilise structurally related molecules which are often dissimilar to their natural substrates. These enzymes are said to exhibit relative group specificity. An example of this kind of enzyme is *Candida cylindracea* (*C*. *cylindracea*) lipase, which will catalyse a transesterification reaction between a variety of acyl donors and acyl acceptors. In addition to chemical specificity, enzymes also exhibit stereochemical specificity.

The International Union of Biochemistry has classified enzymes into six categories according to the type of reaction that they catalyse.

Oxidoreductases catalyse oxidation and reduction reactions. More particularly, they catalyse the oxygenation of C-H, C-C and C=C bonds and the removal or addition of H atom equivalents.

Transferases catalyse the transfer of various groups such as aldehyde, ketone, acyl, sugar, phosphoryl or methyl groups.

Hydrolases catalyse the breakdown of, *inter alia,* esters, amides, lactones, lactams, epoxides, nitriles, anhydrides and glycosides by hydrolysis.

Lyases catalyse the addition-elimination of small molecules onto C=C, C=N and C=O bonds.

Isomerases catalyse isomerisation reactions such as racemisations and epimerisations.

Ligases catalyse the formation and cleavage of C-O, C-S, C-N and C-C bonds with concomitant triphosphate cleavage.

In nature, some enzymes function within or at the lipid layer within a cell membrane. The lipases, for example, are active at the water-lipid interface. The lipid layer provides a non-aqueous and non-polar environment for the working enzyme.

Enzyme catalysts are also used commercially in a number of processes in order to make use of their stereo-selectivity. For example, enzymes of the hydrolase class (proteases and lipases) are used commercially for the resolution of racemic mixtures of secondary alcohols and carboxylic acids, in the conversion of prochiral and centrosymmetric compounds into chiral compounds and in the desymmetrisation of meso compounds. The enzymes operate most effectively in non-polar organic solvents, such as hexane. Increasing the polarity of the solvent tends to result in a rapid deactivation of the enzyme and/or a greatly reduced reaction rate.

It would be desirable to improve upon the commercial enzyme catalysed processes by improving the reaction rate, selectivity and/or conversion to products. It would also be desirable to employ a solvent which is able to dissolve a wide range of reaction substrates, which mitigates the deactivation of the enzyme during the reaction and which allows a given enzyme to be utilised effectively across a wide range of substrates.

In particular, there is a need for an enzyme catalysed process that can stereo-selectively convert a first compound into a second compound more efficiently than the known processes that are in commercial use today.

WO 2004/083444 discloses a process for preparing a second compound stereo-selectively which process comprises reacting a starting material or substrate comprising at least one first compound with a reagent in the presence of a biological catalyst and a solvent comprising at least one (hydro)fluorocarbon.

One disadvantage of this prior art process is that reaction of a racemate to give the desired enantiomeric product selectively has a maximum theoretical yield of 50%. Thus, although the enantiomeric starting material of interest may be completely selectively converted to give the desired enantiomeric product, the unwanted enantiomeric starting material remains unreacted. Not only is this uneconomic, the unwanted starting material must also be separated from the desired product. It is therefore desirable to improve the efficiency of this type of reaction.

A known process is dynamic kinetic resolution (DKR) which uses two reactions in order to improve the efficiency of this type of process. An example of DKR process is given in the document Choi J.H. et al., Angew. Chem. Int. Ed. 2002, vol. 41, no. 41, p.2373 - 2376.

As shown below in Scheme 1, the prior art process discussed above may start with a mixture or racemate of enantiomeric starting materials, shown as R and S. A stereo-selective process can give the desired product P from the starting isomer R in preference to the unwanted product Q from the S isomer. This is due to the selective nature of the catalyst used, with the rate of reaction of the R starting material being much greater than that of the S starting material. Thus, in this prior art process the desired product P may be formed in a maximum of 50% yield, based upon the starting compounds R and S.

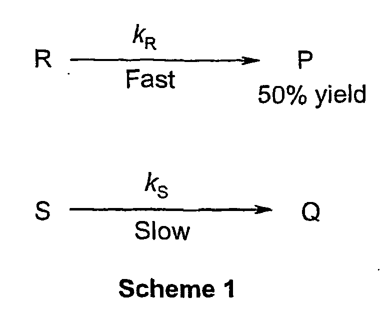

As shown below in Scheme 2, the use of dynamic kinetic resolution may improve on this yield. This process corresponds to the known process shown in Scheme 1 with the addition of a racemisation reaction or equilibration between the R and S starting materials. Thus, as the R starting material is reacted to give the desired product P, the concurrent or simultaneous racemisation reaction forms more of the R starting material from the S starting material. Thus, as the R starting material is removed from the reaction by conversion to the product P, this drives the gradual conversion of the S isomer to the R isomer. Thus, this enables the product P to be formed from a mixture of the S and R starting materials in a theoretical maximum of 100% yield.

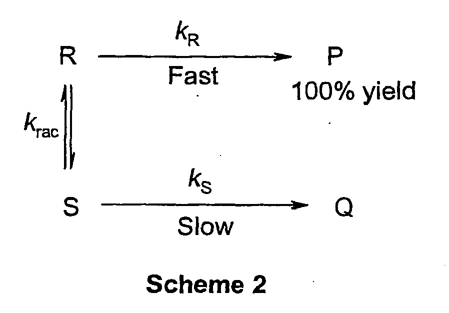

Although examples of the dynamic kinetic resolution reactions are known, they generally suffer from a number of disadvantages. One disadvantage is that the reaction mixtures often use solvents such as toluene. However, solvents such as toluene are not the ideal solvents for use in combination with all desired components of the reaction mixture. Also, it is often necessary to heat the reaction mixture to reflux in solvents such as toluene, with the result that the high temperature either degrades catalysts or prevents the use of the desired catalysts.

Dynamic kinetic resolution reactions often use a racemisation catalyst which comprises a metal or a metal-containing complex, in combination with and a stereo-selective biological catalyst such an enzyme. However, the racemisation catalyst and the biological catalyst often have differing solvent requirements in order to work effectively. For example, the racemisation catalyst may require elevated temperatures in order to function at an acceptable rate, such temperatures being detrimental to the longevity of the enzyme. Similarly, the racemisation catalyst may only have adequate solubility in a solvent in which the enzyme activity is greatly reduced er in which the enzyme is degraded. The solubility and compatibility of the other components of the reaction mixture with the solvent must also be considered, These factors include the solubility and compatibility of the starting materials, any reagents, co-catalysts and co-solvents.

Thus, there exists a need to provide an improved dynamic kinetic resolution process.

According to one aspect of the invention, there is provided a process for preparing a third compound stereo-selectively according to claim 1, which process comprises:
reacting a substrate comprising a first enantiomeric compound with a racemisation catalyst to give a second enantiomeric compound, and concurrently reacting the second enantiomeric compound in the presence of an enzyme to give the third compound, wherein the racemisation catalyst comprises a metal,
wherein the process is performed in a solvent comprising at least one C₁₋₁₀ (hydro) (hydro) fluorocarbon, and wherein the first and second enantiomeric compounds are selected from alcohols, carboxylic acids, carboxylic acid esters, amino acid esters, amines, thiols and amides.

Conveniently, the enzyme is a hydrolase.

Advantageously, the enzyme is selected from the proteases and lipases.

Preferably, the enzyme is part of a whole cell culture.

Advantageously, the first and second enantiomeric compounds are R and S isomers of a compound.

Preferably, the substrate comprises a racemate of the first and second enantiomeric compounds.

Conveniently, the substrate is not a racemate of the first and second enantiomeric compounds.

Conveniently, the at least one C₁₋₁₀ hydrofluorocarbon is selected from the group consisting of difluoromethane (R-32), pentafluoroethane (R-125), 1,1,1-trifluoroethane (R-143a), 1,1,2,2-tetrafluoroethane (R-134), 1,1,1,2-tetrafluoroethane (R-134a), 1,1-difluoroethane (R-152a), 1,1,1,3,3-pentafluoropropane (R-245fa), 1,1,1,2,3,3-hexafluoropropane (R-236ea) and 1,1,1,2,3,3,3-heptafluoropropane (R-227ea), 2,3,3,3-tetrafluoropropene (R-1234yf), 1,3,3,3-tetrafluoropropene (R-1234ze) and (Z)-1,3,3,3-pentafluoropropene (R-1225ye).

Advantageously, the solvent comprises at least one of 1,1,1,2,3,3,3-heptafluoropropane (R-227ea) and 1,1,1,2 tetrafluoroethane (R-134a).

Preferably, the solvent comprises iodofluoromethane (CF₃I).

Advantageously, at least one (hydro)fluorocarbon is used in combination with a co-solvent.

Conveniently, the co-solvent is halogen free.

Advantageously, the solvent is in the liquid state.

Preferably, the process is conducted in the presence of water at a level which is less than that required for the water to form a separate aqueous phase in the reaction system.

Conveniently, the amount of water is used is below the saturation level for the solvent

Advantageously, the amount of water that is used is less than 1% by weight of water based on the total weight of the solvent.

Preferably, the enzyme is *Subtilisin Carlsberg*.

Advantageously, the racemisation catalyst comprises a metal complex.

Preferably, the metal is palladium.

Conveniently, the racemisation catalyst comprises palladium on carbon.

Advantageously, the metal is ruthenium.

Conveniently, the racemisation catalyst comprises a complex of ruthenium (II) and at least one aromatic or heteroaromatic ligand.

Preferably, the racemisation catalyst comprises chlozodicarbanyl[1-(i-propylamino)-2,3,4,5-tetraphenylcyclopentadienyl]ruthenium (II), 1-hydroxytetraphenylcyclopentadienyl(tetraphenyl-2,4-cyclopentadien-1-one)-mu-hydrotetracarbonyldiruthenium (II), or dichloro(p-cymene)ruthenium(II) dimer.

Conveniently, the second enantiomeric compound is reacted with a reagent in the presence of the enzyme to form the third compound.

Advantageously, the reagent is an acyl donor.

Preferably, the reagent is a vinyl alkanoate or an isopropenyl alkanoate.

Advantageously, the reagent is vinyl acetate.

The invention will now be described, by way of example, with reference to the accompanying drawings, in which:
Figure 1 is a graph showing the decrease in enantiomeric excess (ee) of (S)-1-phenyl-2-propanol with racemisation catalyst 3, and
Figure 2 is a graph showing the decrease in enantiomeric excess (ee) of (S)-1-phenyl-1-ethanol with racemisation catalyst 3.

The process of the present invention converts a second compound into a particular chiral third compound or compounds stereo-selectively. By this we mean that the second compound, although capable, in principle, of reacting to form a mixture of stereoisomers, reacts preferentially or selectively under the influence of the biological catalyst to yield predominantly and preferably exclusively one enantiomeric product. In particular, we are referring to a process that yields one particular enantiomeric product predominantly and preferably exclusively. More particularly, the conversion of the starting material or substrate is such that the desired enantiomer is formed at an enantiomeric excess of greater than 50 %, more preferably of greater than 70 % and particularly of greater than 90 %.

The process of the present invention can provide for good conversions of the second compound to the third compound at high stereo-selectivities. The conversions and stereo-selectivities may be better than are obtainable in the known commercial processes that use conventional hydrocarbon solvents such as hexane. Furthermore, the process may proceed at a faster rate and/or at lower temperatures than processes conducted in conventional hydrocarbon or other organic solvents.

It is also believed that the (hydro)fluorocarbon solvent or solvents that are used in the present process may result in less degradation of the enzyme than when the same reaction is conducted using conventional hydrocarbon solvents such as hexane. This, in turn, could allow a continuous process to be run for a longer period of time before changing the catalyst or in a batch process could allow the catalyst to be re-used a greater number of times. This also applies to the racemisation catalyst. Ease of removal of the (hydro)fluorocarbon solvents may assist with facile recovery of catalysts and products and be particularly beneficial for thermally labile materials.

The process of the present invention also involves the conversion of a first compound to the second compound. This means that the first enantiomeric compound is converted into the second enantiomeric compound, using a racemisation catalyst.

The first and second compounds differ by having a chiral centre of opposite configuration. Thus, if the fist compound has a chiral centre of R configuration, the second compound would have the chiral centre with S configuration. The racemisation may proceed directly from the first to second enantiomeric compounds, or may proceed through another compound, such as an achiral or prochiral intermediate. This racemisation process occurs concurrently or simultaneously with the stereo-selective reaction to give the third compound. In general, this means that the reaction is a one-pot process in the presence of the first enantiomeric compound the second enantiomeric compound, the racemisation catalyst, the enzyme, any required reagent and the solvent As the second enantiomeric compound is selectively reacted to form the third compound, the first enantiomeric compound is "pulled through" to the second enantiomeric compound and then onto the desired product. It is readily apparent that the racemisation catalyst need not achieve a completely racemic mixture at any particular instant; only a portion of the unreacted first compound needs to be converted.

The process of the present invention is conducted in the presence of a solvent that comprises at least one C₁₋₁₀ (hydro)fluorocarbon. By the term "(hydro)fluorocarbon" we mean a compound selected from the group consisting of the hydrofluorocarbons, fluoroiodocarbons and the perfluorocarbons. By the term "hydrofluorocarbon" we mean a compound which contains only carbon, hydrogen and fluorine atoms. Hydrofluorocarbons may be saturated, unsaturated, acyclic or aromatic. Hydrofluorocarbon solvents are preferred.

The solvent is usually in the liquid state, although we do not discount the use of supercritical fluids. A supercritical fluid is a fluid at a temperature and pressure above its thermodynamic critical point. Where the solvent comprises one or more low boiling compounds which are gases at room temperature, the desired liquid state may be attained by cooling the solvent to a suitably low temperature and/or by subjecting it to super-atmospheric pressures at some point in the process. One or both of these measures may be applied either before or after the (hydro)fluorocarbon solvent is mixed with the substrate to be reacted and, if necessary, continuously during the process.

Suitable (hydro)fluorocarbons may be selected from the C₁₋₁₀, particularly the C₁₋₅ and especially the C₁₋₄ (hydro)fluorocarbons.

Preferred hydrofluorocarbons are selected from the C₁₋₁₀, particularly the C₁₋₅ and especially the C₁₋₄ hydrofluoroalkanes, and the C₂₋₅ hydrofluoralkenes. Suitable hydrofluorocarbons include hydrofluoromethanes, such as trifluoromethane (R-23), fluoromethane (R-41) and difluoromethane (R-32); hydrofluoroethanes, such as pentafluoroethane (R-125), 1,1,1-trifluoroethane (R-143a), 1,1,2,2-tetrafluoroethane (R-134), 1,1,1,2-tetrafluoroethane (R-134a) and 1,1-difluoroethane (R-152a); hydrofluoropropanes, such as 1,1,1,3,3-pentafluoropropane (R-245fa), 1,1,2,2,3-pentafluoropropane (R-245ca), 1,1,1,2,3-pentafluoropropane (R-245eb), 1,1,2,3,3-pentafluoropropane (R-245ea), 1,1,1,2,3,3-hexafluoropropane (R-236ea), 1,1,1,2,2,3-hexafluoropropane (R-236cb), 1,1,1,3,3,3-hexafluoropropane (R-236fa), 1,1,1,2,3,3,3-heptafluoropropane (R-227ea) and 1,1,1,2,2,3,3-heptafluoropropane (R-227ca); hydrofluoropropenes, including tetrafluoropropenes such as 1,1,1,2-tetrafluoropropene (R-1234yf) and pentafluoropropenes such as (Z)-1,1,1,2,3-pentafluoropropene (R-1225ye); and hydrofluorobutanes, such as 1,1,1,3,3-pentafluorobutane (R-356mfc).

The preferred hydrofluorocarbons are R-32, R-134a, R-134, R-152a, R-143a, R-125, R-245fa, R-236ea and R-227ea, which are all low boiling making their removal from the reaction mixture at the end of the process relatively facile.

Of these, R-227ea and R-134a are particularly preferred.

Fluoroiodocarbons include trifluoroiodomethane and pentafluoroiodoethane. Of these, trifluoroiodomethane is preferred.

Solvents containing mixtures of two or more (hydro)fluorocarbons may be used if desired.

The solvent which is used in the process of the present invention may also comprise an organic co-solvent in addition to the (hydro)fluorocarbon.

Suitable co-solvents include, inter alia, fluorine free and more particularly halogen free compounds. Suitable halogen free co-solvents will typically have a boiling point of 200°C or below, for example in the range of from -85 to 200°C. The preferred co-solvents have a boiling point of 120°C or below, for example in the range of from -85 to 120°C, more preferably 100°C or below, for example in the range of from -70 to 100°C, and particularly 10°C or below, for example in the range of from -60 to 10°C.

Mixtures of two or more co-solvents may be used if desired.

Suitable co-solvents may be selected from the C₂₋₆, particularly the C₂₋₄ hydrocarbon compounds by which we mean compounds containing only carbon and hydrogen atoms. Suitable hydrocarbons include the alkanes and cycloalkanes, with alkanes such as ethane, n-propane, i-propane, n-butane, i-butane and n-pentane being preferred.

Other suitable co-solvents include the hydrocarbon ethers, by which we mean compounds having the formula R¹-O-R² in which R¹ and R² are independently hydrocarbyl groups containing only carbon and hydrogen atoms, such as C₁₋₆ and particularly C₁₋₃ alkyl groups. Suitable dialkyl ethers include dimethyl ether, methyl ethyl ether and diethyl ether.

Still further suitable co-solvents may be selected from the amines, amides, sulphoxides, alcohols, ketones, carboxylic acids, carboxylic acid derivatives, inorganic acids and nitro compounds.

Suitable amines include ammonia, mono-, di-, and tri-alkylamines such as methylamine and triethylamine

Suitable amide co-solvents include the N,N'-dialkylamides and alkylamides, e.g. dimethylformamide and formamide.

Suitable sulphoxide co-solvents include the dialkylsulphoxides, e.g. dimethylsulphoxide.

Suitable alcohol co-solvents include the aliphatic alcohols, particularly the alkanols. Suitable alkanols may be selected from the C₁₋₆, particularly the C₁₋₃ alkanols such as methanol, ethanol, 1-propanol and 2-propanol.

Suitable ketone co-solvents include the aliphatic ketones, particularly the dialkyl ketones such as acetone.

Suitable carboxylic acid co-solvents include formic acid and acetic acid.

Suitable carboxylic acid derivatives for use as co-solvents include the anhydrides, e.g. acetic anhydride, and the C₁₋₆, particularly the C₁₋₃ alkyl esters of C₁₋₆, particularly C₁₋₃ alkanoic acids, e.g. ethyl acetate.

Suitable nitro compounds for use as co-solvents include the nitroalkanes and nitroaryl compounds, e.g. nitromethane and nitrobenzene.

Although not preferred, when an organic co-solvent is used the solvent blend will typically comprise from 80.0 to 99.0 % by weight of the (hydro)fluorocarbon and from 1 to 20 % by weight of the co-solvent. Preferably, the solvent blend will comprise from 90.0 to 99.0 % by weight of the (hydro)fluorocarbon and from 1 to 10.0 % by weight of the co-solvent. As the polarity of the co-solvent is increased, it is generally desired to use less of the co-solvent in order to avoid any problems with deactivation of the enzyme.

Preferably, no water is added to the reactions. However, the process of the present invention may be conducted in the presence of at least a small amount of water. The amount of water that may be used will usually be such that the water does not form a separate phase in the reaction system. This is because an objective of the present process is to have the enzyme function in an environment that is predominantly composed of the (hydro)fluorocarbon solvent. Preferably, the amount of water is kept below the saturation level of the solvent that is used. More preferably, the reaction is conducted in the presence of less than 1 % by weight of water based on the total weight of the solvent. The absence of free-phase water is of particular benefit in cases where the substrates, products or racemisation catalysts are water sensitive.

The process of the present invention is conducted in the presence of an enzyme. The enzyme must, of course, be capable of catalysing a stereo-selective conversion of the second compound into the third compound. In certain circumstances, it may be beneficial or necessary to include additional components such as cofactors.

Typically, the process of the present invention will be conducted in the presence of a single enzyme, although we do not discount the possibility that mixtures of catalysts may be used.

Suitable enzymes for use in the present process may be selected from any of the six classes of enzymes which have been identified *supra*.

The enzymes may be discrete in the sense that they have been isolated from the biological tissue in which they normally reside or else produced by over-expression in a host organism. These discrete enzymes may be used as they are or they may be lyophilised using standard literature processes, e.g. as described in Fitzpatrick, P. A., Klibanov, A. M., J. Am. Chem. Soc., 1991, 113, 3166. However, we have found that at least some enzymes are able to function as effectively in a (hydro)fluorocarbon solvent without prior lyophilisation, therefore offering the potential of avoiding a significant processing step.

The enzymes, whether lyophilised or not, are usually immobilised using standard literature processes. For example, the enzyme may be immobilised on a solid, insoluble matrix, for example by physical absorption or bonding. Suitable matrices include, *inter alia*, glass, diatomaceous earth, silica and organic polymers such as polystyrene and polyacrylate homopolymers and copolymers.

The enzymes may be used without being immobilised.

Alternatively, the enzymes may be part of a whole cell culture such as a live cell culture, e.g. Lactobacillus acidophilus, a resting cell culture, e.g. dried baker's yeast which can be activated by warm water or a non-viable cell culture which contains the enzyme and the required cofactor(s), e.g. dead yeast. The whole cell culture containing the enzyme will usually be immobilised on a solid, insoluble matrix, for example by physical absorption or bonding, using standard literature processes. The matrices discussed above may be used for this purpose. The cultures may be used without being immobilisation.

Preferred enzymes for use in the process of the present invention include those in the hydrolase category. Particular enzymes are the proteases, such as *Subtilisin carlsberg* and *Subtilisin BPN,* the lipases, such as *Novozym 435, Porcine pancreatic lipase, Candida antarctica B lipase* and *Pseudomonas cepacia lipase* and the glycosidases such as α- and β-galactosidase from *Aspergillus orgzea*.

The process of the present invention is conducted in the presence of a racemisation catalyst. By a "racemisation catalyst" we mean a catalyst that is able to transform a first enantiomeric compound to a second enantiomeric compound by inversion of the configuration of a chiral centre. This conversion may be direct or may proceed via an intermediate compound, such as an achiral or prochiral compound. Preferred racemisation catalysts comprise a metal, such as transition metal. In particular, racemisation catalysts comprising ruthenium (II) complexes are preferred. Particularly preferred are Ru (II) complexes comprising substituted aromatic or heteroaromatic hydrocarbon ligands.

The three ruthenium catalysts shown below are preferred.

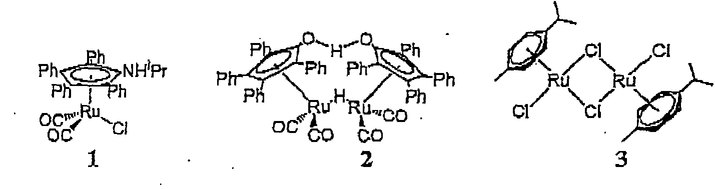

Racemisation catalyst 1 is chlorodicarbonyl[1-(i-propylamino)-2,3,4,5-tetraphenylcyclopentadienyl]ruthenium (II), racemisation catalyst 2 is 1-hydroxytetraphenylcyclopentadienyl(tetraphenyl-2,4-cyclopentadien-1-one)-mu-hydrotetracarbonyldiruthenium (II), and racemisation catalyst 3 is dichloro(p-cymene)ruthenium (II) dimer.

The racemisation catalyst is typically used in larger amounts than the biological catalyst, for example, the racemisation catalyst is used in the range of 0.01 to 30 mole %, preferably 2 to 20 mole %, and more preferably 4 to 10 mole % relative to the substrate.

The process is generally conducted at a temperature which provides for an acceptable rate of reaction and component solubility and which avoids significant degradation of the components of the reaction mixture, including the biological catalyst, the racemisation catalyst, the first compound(s), second compound(s) and the third compound(s). Typically, the process is conducted at a temperature in the range of from -60 to 120°C, preferably in the range of from -30 to 80°C and particularly in the range of from 0 to 60°C, for example at about 20°C.

The process may be conducted at atmospheric, sub-atmospheric or super-atmospheric pressures. The precise operating pressure will depend, inter alia, on the solvent that is used, particularly its boiling point. Preferred absolute operating pressures are in the range of from 0.1 to 200 bar, more preferably in the range of from 0.5 to 30 bar and particularly in the range of from 1 to 15 bar.

The weight ratio of the (hydro)fluorocarbon solvent to the substrate to be reacted is preferably in the range of from 1:1 to 1000:1, more preferably in the range of from 1:1 to 500:1 and particularly in the range of from 1:1 to 10:1. The biological catalyst is typically used very small amounts, for example of the order of 10⁻³ to 10⁻⁴ mole % of catalyst relative to the substrate. The precise amount will depend on the activity of the enzyme.

The process of the present invention can be usefully applied to various stereo-selective conversions. It is particularly useful for preparing compounds that can be used as intermediates or final products in the manufacture of pharmaceutical, flavour and fragrance, insect control, and plant disease control compositions.

In one embodiment, the process of the present invention is used to resolve a racemic mixture by reacting that mixture with a reagent in the presence of the enzyme and (hydro)fluorocarbon solvent so as to preferentially or selectively react one of the enantiomers forming the racemic mixture to form a new enantiomeric compound while leaving the other enantiomer largely or completely unreacted. This unreacted enantiomer is racemised by the racemisation catalyst to form the enantiomer that does react with the enzyme. Thus, as the desired enantiomer is formed from the unwanted enantiomer, the enzyme selectively reacts with the desired enantiomer to give the enantiomeric product.

The racemic mixture comprises R and S enantiomeric isomers of a starting material. Preferably, the R and S isomers are initially present in equal amounts. Alternatively, the isomers may be present in unequal amounts.

Accordingly, in one embodiment of the present invention there is provided a process of resolving a racemic mixture which process comprises reacting the mixture with a reagent in the presence of an enzyme and a solvent comprising at least one (hydro)fluorocarbon so as to preferentially or selectively convert one of the enantiomers forming the racemic mixture into a new enantiomeric compound. The process is performed in the presence of a racemisation catalyst which converts the unreacted enantiomeric starting compound to the preferred enantiomeric starting compound. This, the unwanted enantiomeric starting compound is converted to the desired enantiomeric product via the preferred enantiomeric starting compound.

The racemic mixture that is resolved in accordance with this embodiment of the present invention may comprise R and S alcohols, R and S carboxylic acids or esters, R and S amino acid esters, R and S amines, R and S thiols or R and S amides. Preferably, the mixture comprises R and S amino-acid esters. This particular resolution is effected by preferentially or selectively transforming a functional group attached to the chiral carbon(s) of either the R or S enantiomer. The racemisation catalyst preferably comprises ruthenium.

In one embodiment, the process of the present invention is used to resolve racemic 1-phenylethanol, by a transesterification reaction in which the OH group of either the R or S enantiomer is exchanged preferentially and preferably selectively by reaction with a reagent. The reagent that is used is preferably an acyl donor, e.g. an enol ester, such as a vinyl or isopropenyl alkanoate, or an alkoxy enol ester. The preferred reagent is vinyl acetate. Ordinarily, it is the R enantiomer that undergoes the transesterification. The enzyme is preferably a lipase, for example *Candida antarctica* B Lipase.

The molar ratio of the 1-phenylethanol to the acyl donor is preferably in the range of from 1:0.1 to 1:100, more preferably in the range of from 1:1 to 1:50, for example 1:20.

The reaction time is typically in the range of from 0.1 to 48 hours, preferably in the range of from 1 to 36 hours and particularly in the range of from 1 to 24 hours.

The preferential/selective transesterification of the R or S enantiomer (normally the R enantiomer) of the racemic 1-phenylethanol is such that the desired enantiomer is typically formed at an enantiomeric excess of greater than 50 %, preferably of greater than 70 % and particularly of greater than 90 %, e.g. 100 %.

The resolution of the racemic 1-phenylethanol without concurrent racemisation using vinyl acetate and assuming a 100 % enantiomeric excess of the R enantiomer is shown in Scheme 3.

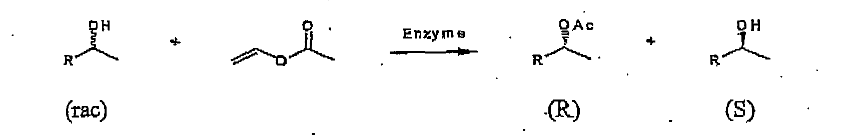

In accordance with one embodiment of the present invention, the above reaction is performed with in situ racemisation, i.e. dynamic kinetic resolution. The process is shown below in Scheme 4.

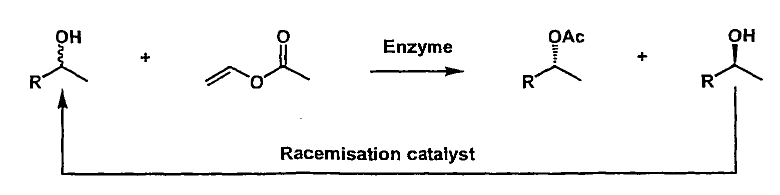

The dynamic kinetic resolution reaction in Scheme 4 corresponds to that shown above in Scheme 3 above except that the unreacted S alcohol is at least partially racemised to give some of the R isomer which reacts with the biological catalyst to give the desired product. In contrast to the reaction shown above in Scheme 3, the dynamic kinetic resolution reaction shown in Scheme 4 may increase the theoretical maximum yield from 50% to 100%, whilst retaining the theoretical maximum ee of 100%.

The process of the present invention may be operated in batch mode or continuously. Where a (hydro)fluorocarbon solvent that has a boiling point below ambient is used, the reaction vessel will typically be a pressure vessel that is capable of withstanding elevated pressures.

In the batch process, the (hydro)fluorocarbon solvent is removed at the end of the process, e.g. by flash evaporation if the (hydro)fluorocarbon is a gas at ambient temperatures or by distillation, to yield a crude reaction mixture which can then be purified, if required, to isolate the desired product.

In a continuous process, a reactant stream comprising the (hydro)fluorocarbon solvent and the reactants is conveyed continuously through a reaction vessel containing the biological catalyst and the racemisation catalyst. The racemisation catalyst may be at least in part dissolved in the (hydro)fluorocarbon solvent. Typically, the reactant stream is passed over a bed of immobilised catalyst(s). The crude reaction mixture that exits the reaction vessel is then treated, e.g. in a solvent evaporator, to remove the (hydro)fluorocarbon solvent and recover the desired product that has been formed in the process. The (hydro)fluorocarbon solvent that has been removed can be condensed and recycled if desired to minimise solvent use. Unreacted starting material and racemisation catalyst may also be recycled if desired.

Where solvent is to be recycled, a suitable recovery system for low boiling point solvents, by which we mean solvents having a boiling point of 25°C or below, e.g. 0°C or below, comprises an evaporator into which the crude reaction mixture emerging from the process is passed, a compressor for compressing the vapour generated in the evaporator and a condenser for cooling the compressed vapour emerging from the compressor. The solvent is removed from the crude reaction mixture in the evaporator by flash evaporation induced by suction from the compressor and the solvent vapour so generated then passes to the compressor, which may be a diaphragm compressor, where it is compressed. From the compressor, the solvent vapour passes to the condenser where it is cooled and returned to liquid form for recharging to the process or possibly to a solvent reservoir supplying solvent to the process. The condenser, which may take the form of a coiled tube, can be arranged inside the evaporator so that the latent heat of condensation provides at least some of the energy required to evaporate the solvent.

A further suitable recovery system for low boiling point solvents comprises a solvent recycling circuit comprising an evaporator into which the reaction mixture emerging from the process is passed and in which the solvent is evaporated and a condenser in which the vapour emerging from the evaporator is cooled and returned to liquid form for recharging to the process or possibly to a solvent reservoir supplying solvent to the process. Heating of the evaporator and cooling of the condenser may be carried out independently, but in a preferred embodiment an external heat pump system is used to both heat the evaporator and to cool the condenser. The external heat pump system comprises .an evaporator, a compressor, a condenser and an expansion valve which are sequentially arranged in a circuit through which a heat transfer fluid is caused to flow. The evaporator of the external heat pump system, which may take the form of a coiled tube, is arranged inside or around the outside of the condenser of the solvent recycling circuit so that evaporation of the heat transfer fluid in the evaporator cools the condenser and provides for the condensation of the solvent vapour passing through the solvent recycling circuit. The vapour generated in the evaporator of the external heat pump system is then compressed and passes to the condenser where it condenses and gives off heat. The condenser of the external heat pump system, which may also take the form of a coiled tube, is arranged inside or around the outside of the evaporator of the solvent recycling circuit so that the latent heat of condensation associated with the condensation of the heat transfer fluid provides the heat required to evaporate the solvent passing through the solvent recycling circuit. The condensed heat transfer fluid is then returned through an expansion valve to the evaporator so completing the cycle in the external heat pump system.

As an alternative to an external heat pump system, an external circulating heat-transfer fluid may be used to transfer the heat of solvent condensation to the evaporator vessel to provide heat for solvent evaporation.

When the process of the present invention is complete, the crude reaction mixture may be subjected to a purification step in order to isolate the desired product. The pure product may then be subjected to one or more further synthetic steps, e.g. to yield a pharmaceutical compound. Alternatively, the crude reaction mixture may be used directly in a further synthesis. Suitable purification techniques include those that are routinely used in chemical synthesis such as chromatography, crystallisation and distillation.

The present invention is now illustrated but not limited by the following examples.

### General Procedures

R-134a and R-227ea were supplied by Ineos Fluor Ltd. and used without further purification. Both solvents were maintained in the liquid state under autogenous pressure by conducting the reaction in suitable vessels such as standard plastic-coated 10 ml glass aerosol bottles.

The enzymes were obtained from Aldrich Chemical Company or Sigma Chemical Company. All other chemicals and solvents were purchased from Aldrich Chemical Company and used without further purification.

Gas chromatograms were recorded using a Shimadzu GC-17a instrument equipped with an HP SE-54 capillary column (25m x 0.21mm i.d.). Chiral gas chromatograms were obtained on a Chrompack CP9001 instrument fitted with a Chiraldex GTA capillary column (30m x 0.25mm i.d.). Flame ionisation detectors were used in both cases and response factors calibrated for individual substances using standard solutions. The samples that were removed from the mixture were taken up in dichloromethane.

Chiral liquid chromatograms were recorded on a Hewlett Packard 1050 series, equipped with a Chiralcel OD analytical column (250 x 4.6mm ID)

### Dynamic Kinetic Resolution.

As mentioned above, kinetic resolutions have been used for many years, in a variety of different media, to obtain enantiopure materials. However one of the drawbacks of kinetic resolutions is the yield limitations they present. In a traditional kinetic resolution, only one enantiomer is used, and the other goes to waste or, at best, can be used in a different process. Dynamic kinetic resolutions solve this problem by racemising the unwanted enantiomer *in situ* whilst the kinetic resolution is occurring. This racemisation is often achieved using a metal based catalyst, often ruthenium or palladium. One of the problems in applying this technique to enzymatic kinetic resolutions is finding a solvent suitable for both the enzyme and the racemisation catalyst. (Hydro)fluorocarbon solvents such as R-134a, R-227ea and R-32 appear to show compatibility with both the racemisation catalysts and the enzymes, both of which show comparable or enhanced activities in the (hydro)fluorocarbons as compared to more commonly used solvents.

### Racemisations

The three racemisation catalysts 1, 2, 3 shown above were used to perform dynamic kinetic resolutions.

The three racemisation catalysts 1, 2, 3 were used to racemise enantiomerically pure (S)-1-phenylethanol 4 in R-134a (Scheme 5). This was compared against the racemisation under identical conditions in toluene, the solvent often used for prior art dynamic kinetic resolutions. For all three catalysts, there is a considerable increase in racemisation in the (hydro)fluorocarbon, most notably with racemisation catalyst 1 (Table 1).

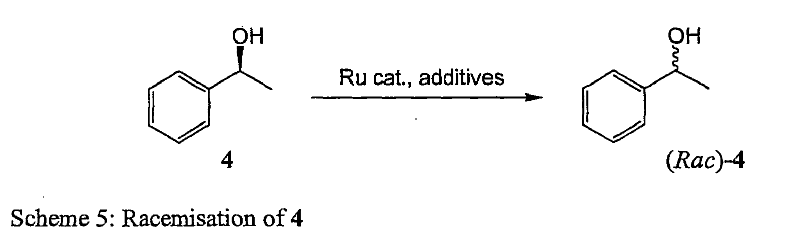

**Table 1: Racemisation of (S)-1-phenyl ethanol 4 with different racemisation catalyst Ru catalysts after 24h at room temperature. ^{a}1.5mmol 4, 1.5mmol Na₂CO₃, 0.08mmol ^{t}BuOK, 4mol % racemisation catalyst 1, ^{b}0.5mmol (S)-1-phenyl ethanol 4, 6mol % racemisation catalyst 2, ^{c}1.5mmol (S)-1-phenyl ethanol 4, 1.5mmol NEt₃, 21mol %racemisation catalyst 3**

| | **e.e. (%)** | | |
|---|---|---|---|
| **Solvent** | **1**^{a} | **2**^{b} | **3**^{c} |
| R-134a | 3.50 | 33.70 | 8.4 |
| toluene | 58.10 | 75.20 | 32.0 |

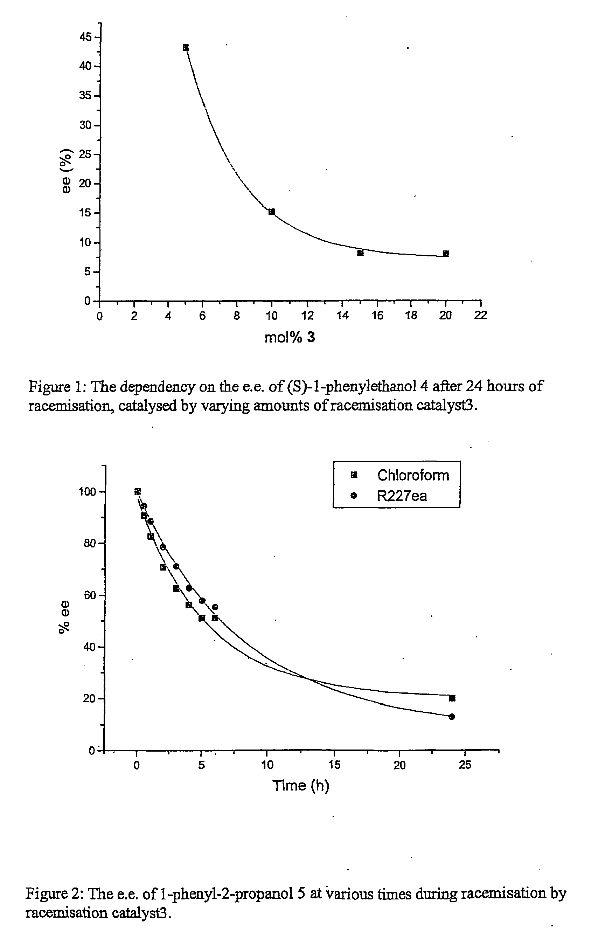

The amount of catalyst required to maintain the rate of racemisation of the S alcohol 4 by catalyst 1 has also been studied, and the difference between 15 mol% and 20 mol% of the catalyst appears to be negligible. The results are shown in Figure 1. It can be seen that catalyst 1 effectively racemises the S alcohol 4 within a few hours.

A more thorough study has been conducted into the racemisation of (S)-1-phenyl-2-propanol 5, catalysed by racemisation catalyst 3 (Scheme 6). The racemisation was performed in the seven solvents listed in Table 2, namely R-227ea, chloroform, R-134a, vinyl acetate, toluene, hexane and tert-butyl methyl ether (TBME), with R-227ea appearing to be the best solvent, and the catalyst in R-134a showing the third highest activity. The only organic solvent in which the catalyst shows comparable activity is chloroform (Table 2). However, chloroform is not an attractive choice of solvent for DKR reactions because of degradation of many enzymes and increasing regulatory pressure on residual levels of chloroform in products in the pharmaceutical and other fine chemical sectors.

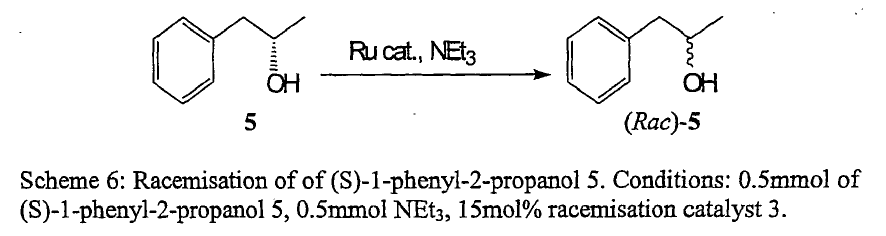

**Table 2: Racemisation of (S)-1-phenyl-2-propanol 5 in different solvents. Conditions: 0.5mmol of (S)-1-phenyl-2-propanol 5, 0.5mmol NEt₃, 15mol% racemisation catalyst 3, after 24h at room temperature.**

| **Solvent** | **e.e. (%)** |
|---|---|
| R-227ea | 4.48 |
| chloroform | 10.06 |
| R-134a | 15.10 |
| vinyl acetate | 65.94 |
| toluene | 75.96 |
| hexane | 86.26 |
| TBME | 90.38 |

The racemisation of (S)-1-phenyl-2-propanol 5 by racemisation catalyst 3 was followed more closely in R-227ea and chloroform. This study indicated that the initial rate of racemisation was greater in chloroform, but that there was a crossover in the rates, indicating greater longevity of the catalyst in the HFC. The results are shown in Figure 2.

### Dynamic kinetic resolutions

As the results discussed both show that the racemisation catalysts have increased activity in (hydro)flnorocarbons, they were used in dynamic kinetic resolutions. The ruthenium catalysis 3 and 4 that have been used in the racemisation studies were used in combination with the commercially available biological catalyst Novozym 435 RTM for dynamic kinetic resolutions of secondary alcohols in R-134a.

One reaction studied was the dynamic kinetic resolution of 2-chloro-1-phenylethanol 6. The results showed that the desired enantiomeric product 8 could be obtained in 72% yield and 80% ee, demonstrating that dynamic kinetic resolutions can be performed in (hydro)fluorocarbons (Scheme 7).

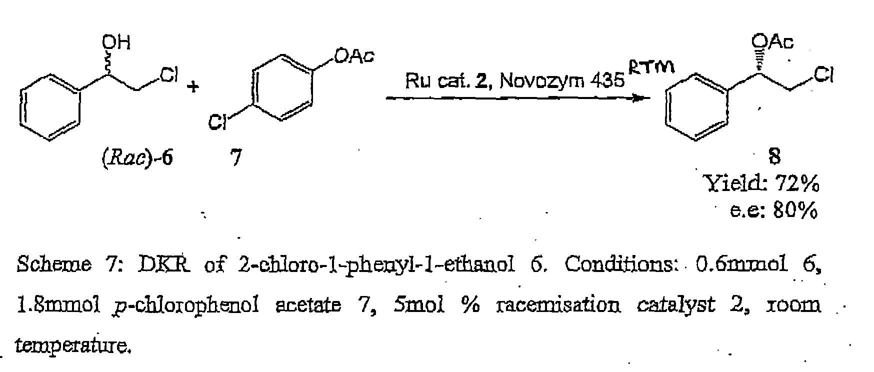

Dynamic kinetic resolutions have also been performed on the alcohol 1-phenyl ethanol 4, using ruthenium catalyst 3 (Scheme 8). The enzyme, substrate and racemisation catalyst levels were varied, leading to 90+ % yield and e.e. being achieved (Table 3).

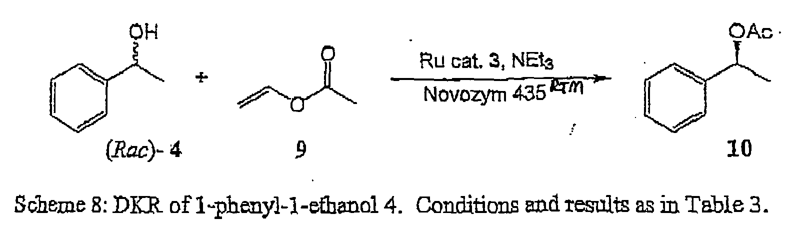

**Table 3: Optimization of DKR of 4 at room temperature.**

| 4 (mM) | 9 (mM) | **Enzyme** (U) | mol % 3 | **Yield** (%) | ee (%) |
|---|---|---|---|---|---|
| 100 | 2000 | 100 | 10.7 | 68.1 | 90 |
| 100 | 2000 | 10 | 20.8 | 81.7 | 82 |
| 100 | 1000 | 10 | 21.2 | 86.1 | 83 |
| 200 | 1000 | 10 | 10.5 | 82.3 | 81 |
| 200 | 1000 | 10 | 20.0 | 88.1 | 85 |
| 100 | 500 | 10 | 20.9 | 87.6 | 86 |
| 300 | 900 | 4500 | 20.6 | 78.2 | 91 |
| 100 | 300 | 1500 | 21.1 | 80.5 | 91 |
| 300 | 900 | 2025 | 21.1 | 97.8 | 81 |
| 300 | 300 | 2025 | 21.1 | 92.3 | 86 |
| 300 | 900 | 4500 | 20.6 | 95.1 | 93 |

As can be seen in the dynamic kinetic resolution of racemic 1-phenyl-2-propanol the biological catalyst Novozym 435 RTM also shows good selectivity under these conditions (Scheme 9).

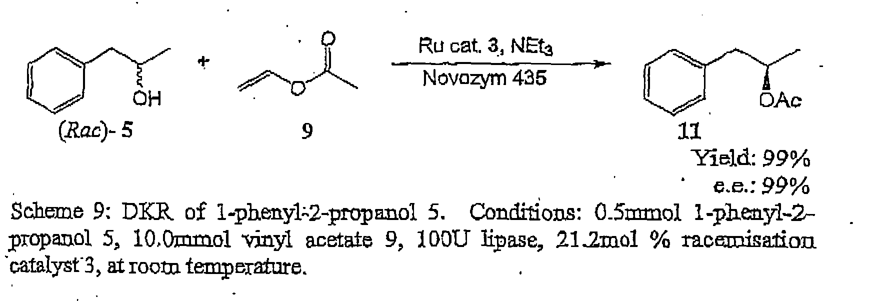

Other points to note about the dynamic kinetic resolutions of the present invention are the short reaction times, the use of mild and convenient reagents such as vinyl acetate and the fact that the reactions may be done at ambient temperature. Prior art dynamic kinetic resolutions can be long processes involving high temperatures, using refluxing toluene as the solvent, and may use expensive, not readily available acyl donors.

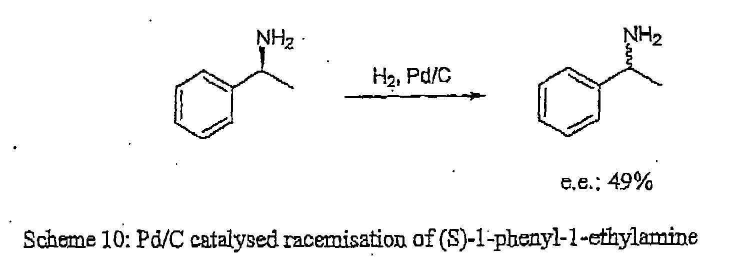

Scheme 10 shows that palladium on carbon may be used to racemise (S)-1-phenyl-1-ethylamine, which process may be used in DKR reactions of the present invention.

### Preparation of (S)-1-phenyl-2-propanol

To a 1L metal cylinder was added (±)-1-phenyl-2-propanol (16.3g, 120mmol). To this was added vinyl acetate (30mL, 325mmol) and Novozym 435 RTM (1.2g, 13440U). The vessel was sealed and charged with R-134a (130g, 100mL). The vessel was shaken at 160rpm at 25°C for 72h. The R-134a was vented, and the supported enzyme filtered from the crude product. The crude product was purified by flash chromatography (dichloromethane) to give (*S*)-1-phenyl-2-propanol as an oil (94% yield, 99% e.e.)

### Racemisation of (S)-1-phenyl-1-ethanol using racemisation catalyst 1

1.5mmol (*S*)-1-phenyl-1-ethanol, 1.5mmol Na₂CO₃, 0.08mmol ^{t}BuOK, and 4mol % racemisation catalyst 1 were placed in a flask. To this was added toluene (5mL). The vessel was sealed, and the mixture stirred at room temperature. After 24h, the mixture was sampled and analysed by gas chromatography for enantiomeric excess.

The same reaction was investigated using R-134a as the solvent. A mixture of 1.5mmol (*S*)-1-phenyl-1-ethanol, 1.5mmol Na₂CO₃, 0.08mmol ^{t}BuOK, and 4mol % racemisation catalyst 1 was prepared in a glass aerosol bottle. The aerosol bottle was then capped, and the cap crimped in place. A weighed quantity of liquid R-134a (6.50g, 5mL) was introduced through the aerosol valve from a larger pressure vessel. The resultant mixture was then stirred at room temperature. After 24 hours the mixture was sampled by venting a small portion of the reaction mixture through the aerosol valve. The R-134a evaporated in the process, leaving behind the low volatility residue of the reaction mixture. This was taken up in dichloromethane, and analysed by gas chromatography for enantiomeric excess.

### Racemisation of (S)-1-phenyl-1-ethanol using racemisation catalyst 2

0.5mmol (*S*)-1-phenyl-1-ethanol, and 6mol % racemisation catalyst 2 were placed in a flask. To this was added toluene (5mL). The vessel was sealed, and the mixture stirred at room temperature. After 24h, the mixture was sampled and analysed by gas chromatography for enantiomeric excess.

The same reaction was investigated using R-134a as the solvent. A mixture of 0.5mmol (*S*)-1-phenyl-1-ethanol, and 6mol % racemisation catalyst 2 was prepared in a glass aerosol bottle. The aerosol bottle was then capped, and the cap crimped in place. A weighed quantity of liquid R-134a (6.50g, 5mL) was introduced through the aerosol valve from a larger pressure vessel. The resultant mixture was then stirred at room temperature. After 24 hours the mixture was sampled by venting a small portion of the reaction mixture through the aerosol valve. The R-134a evaporated in the process, leaving behind the low volatility residue of the reaction mixture. This was taken up in dichloromethane, and analysed by gas chromatography for enantiomeric excess.

### Racemisation of (S)-1-phenyl-1-ethanol using racemisation catalyst 3

1.5mmol (*S*)-1-phenyl-1-ethanol, 1.5mmol NEt₃, 21mol % 3 were placed in a flask. To this was added toluene (5mL). The vessel was sealed, and the mixture stirred at room temperature. After 24h, the mixture was sampled and analysed by gas chromatography for enantiomeric excess.

The same reaction was investigated using R-134a as the solvent. A mixture of 1.5mmol (*S*)-1-phenyl-1-ethanol, 1.5mmol NEt₃, and 21mol % racemisation catalyst 3 was prepared in a glass aerosol bottle. The aerosol bottle was then capped, and the cap crimped in place. A weighed quantity of liquid R-134a (6.50g, 5mL) was introduced through the aerosol valve from a larger pressure vessel. The resultant mixture was then stirred at room temperature. After 24 hours the mixture was sampled by venting a small portion of the reaction mixture through the aerosol valve. The R-134a evaporated in the process, leaving behind the low volatility residue of the reaction mixture. This was taken up in dichloromethane, and analysed by gas chromatography for enantiomeric excess.

### Optimisation of racemisation of (S)-1-phenyl-1-ethanol using racemisation catalyst 3

A mixture of 1.5mmol (*S*)-1-phenyl-1-ethanol, 1.5mmol NEt₃ and racemisation catalyst 3 (varying amounts, see Table 3) was prepared in a glass aerosol bottle. The aerosol bottle was then capped, and the cap crimped in place. A weighed quantity of liquid R-134a (6.50g, 5mL) was introduced through the aerosol valve from a larger pressure vessel. The resultant mixture was then stirred at room temperature. After 24 hours the mixture was sampled by venting a small portion of the reaction mixture through the aerosol valve. The R-134a evaporated in the process, leaving the low volatility residue of the reaction mixture. This was taken up in dichloromethane, and analysed by gas chromatography for enantiomeric excess.

### Racemisation of (S)-1-phenyl-2-propanol using racemisation catalyst 3

Mixtures of 0.5mmol (*S*)-1-phenyl-1-ethanol, 0.5mmol NEt₃, and 15mol % racemisation catalyst 3 were prepared in each of the solvents toluene, chloroform, hexane, TBME and vinyl acetate (5mL). The vessel was sealed, and the mixture stirred at room temperature. After 24h, the mixtures were sampled and analysed by gas chromatography for enantiomeric excess.

The same reaction was investigated using R-134a and R-227ea as the solvents. Mixtures of 0.5mmol (*S*)-1-phenyl-2-propanol, 0.5mmol NEt₃, and 15mol % racemisation catalyst 3 were prepared in glass aerosol bottles. The aerosol bottles were then capped, and the caps crimped in place. A weighed quantity of liquid R-134a (6.50g, 5mL) or R-227ea (6.95g, 5mL) were introduced through the aerosol valves from a larger pressure vessel. The resultant mixtures were then stirred at room temperature. After 24 hours the mixtures was sampled by venting a small portion of the reaction mixtures through the aerosol valves. The (hydro)fluoro carbon solvents evaporated in the process, leaving behind the low volatility residue of the reaction mixtures. These were taken up in hexane, and analysed by HPLC for enantiomeric excess.

### Pd/C catalysed racemisation of (S)-1-phenyl-1-ethylamine (Scheme 10)

A mixture of 0.33mmol (*S*)-1-phenyl-1-ethylamine, and 10% Pd/C (40mg) was prepared in a glass aerosol bottle. The aerosol bottle was then capped, and the cap crimped in place. The flask was then evacuated to form a vacuum, and filled with hydrogen gas. A weighed quantity of liquid R-134a (6.50g, 5mL) was introduced through the aerosol valve from a larger pressure vessel. The resultant mixture was then stirred at 35°C. After 24 hours the mixture was sampled by venting a small portion of the reaction mixture through the aerosol valve. The R-134a evaporated in the process, leaving behind the low volatility residue of the reaction mixture. This was taken up in hexane, and analysed by HPLC for enantiomeric excess.

### Dynamic kinetic resolution of 2-chloro-1-phenyl-1-ethanol

A mixture of 0.6mmol 2-chloro-1-phenyl-1-ethanol, 1.8mmol *p*-chlorophenol acetate, 5mol % racemisation catalyst 2 and 36U of Novozym 435 RTM was prepared in a glass aerosol bottle. The aerosol bottle was then capped, and the cap crimped in place. A weighed quantity of liquid R-134a (6.50g, 5mL) was introduced through the aerosol valve from a larger pressure vessel. The resultant mixture was then stirred at room temperature. After 24 hours the mixture was sampled by venting a small portion of the reaction mixture through the aerosol valve. The R-134a evaporated in the process, leaving behind the low volatility residue of the reaction mixture. This was taken up in dichloromethane, and analysed by gas chromatography for yield and enantiomeric excess.

### Dynamic kinetic resolution of 1-phenyl-1-ethanol

A mixture of 1.5mmol 1-phenyl-1-ethanol, 4.5mmol vinyl acetate, 1.5mmol NEt₃, 20.6mol % racemisation catalyst 3 and 4500U of Novozym 435 was prepared in a glass aerosol bottle. The aerosol bottle was then capped, and the cap crimped in place. A weighed quantity of liquid R-134a (6.50g, 5mL) was introduced through the aerosol valve from a larger pressure vessel. The resultant mixture was then stirred at room temperature. After 24 hours the mixture was sampled by venting a small portion of the reaction mixture through the aerosol valve. The R-134a evaporated in the process, leaving behind the low volatility residue of the reaction mixture. This was taken up in dichloromethane, and analysed by gas chromatography for yield and enantiomeric excess.

### Dynamic kinetic resolution of 1-phenyl-2-propanol

A mixture of 0.5mmol 1-phenyl-1-propanol, 10.0mmol vinyl acetate, 0.5mmol NEt₃, 21.2mol % racemisation catalyst 3 and 100U of Novozym 435 RTM was prepared in a glass aerosol bottle. The aerosol bottle was then capped, and the cap crimped in place. A weighed quantity of liquid R-134a (6.50g, 5mL) was introduced through the aerosol valve from a larger pressure vesseL The resultant mixture was then stirred at room temperature. After 48 hours the mixture was sampled by venting a small portion of the reaction mixture through the aerosol valve. The R-134a evaporated in the process, leaving behind the low volatility residue of the reaction mixture. This was taken up in hexane, and analysed by gas chromatography for yield and HPLC for enantiomeric excess.

## Claims

1. A process for preparing a third compound stereo-selectively which process comprises:
reacting a substrate comprising a first enantiomeric compound with a racemisation catalyst to give a second enantiomeric compound, and concurrently reacting the second enantiomeric compound in the presence of an enzyme to give the third compound,
wherein the racemisation catalyst comprises a metal,
wherein the process is performed in a solvent comprising at least one C₁₋₁₀ (hydro) (hydro) fluorocarbon, and
wherein the first and second enantiomeric compounds are selected from alcohols, carboxylic acids, carboxylic acid esters, amino acid esters, amines, thiols, and amides.

2. A process according to Claim 1 wherein the enzyme is a hydrolase.

3. A process according to Claim 2 wherein the enzyme is selected from the proteases and lipases.

4. A process according to any of Claims 1 to 3 wherein the enzyme is part of a whole cell culture.

5. A process according to any preceding claim wherein the first and second enantiomeric compounds are R and S isomers of a compound.

6. A process according to any preceding claim wherein the substrate comprises a racemate of the first and second enantiomeric compounds.

7. A process according to any one of Claims 1 to 5 wherein the substrate is not a racemate of the first and second enantiomeric compounds.

8. A process according to any preceding Claim wherein the at least one C₁₋₁₀ hydrofluorocarbon is selected from the group consisting of difluoromethane (R-32), pentafluoroethane (R-125), 1,1,1-trifluoroethane (R-143a), 1,1,2,2-tetrafluoroethane (R-134), 1,1,1,2-tetrafluoroethane (R-134a), 1,1-difluoroethane (R-152a), 1,1,1,3,3-pentafluoropropane (R-245fa), 1,1,1,2,3,3-hexafluoropropane (R-236ea) and 1,1,1,2,3,3,3-heptafluoropropane (R-227ea), 2,3,3,3-tetrafluoropropene (R-1234yf), 1,3,3,3-tetrafluoropropene (R-1234ze) and (Z)-1,2,3,3,3-pentafluoropropene (R-1225ye).

9. A process according to Claim 8 wherein the solvent comprises at least one of 1,1,1,2,3,3,3-heptafluoropropane (R-227ea) and 1,1,1,2 tetrafluoroethane (R-134a).

10. A process according to any of Claims 1 to 7 wherein the solvent comprises iodotrifluoromethane (CF₃I).

11. A process according to any preceding claim wherein the at least one (hydro)fluorocarbon is used in combination with a co-solvent.

12. A process according to Claim 11 wherein the co-solvent is halogen free.

13. A process according to any preceding claim wherein the solvent is in the liquid state.

14. A process according to any preceding claim which s conducted in the presence of water at a level which is less than that required for the water to form a separate aqueous phase in the reaction system.

15. A process according to Claim 14 wherein the amount of water is used is below the saturation level for the solvent.

16. A process according to Claim 14 or 15 wherein the amount of water that is used is less than 1% by weight of water based on the total weight of the solvent.

17. A process according to any preceding claim wherein the enzyme is *Subtilisin Carlsberg.*

18. A process according to any preceding claim wherein the racemisation catalyst comprises a metal complex.

19. A process according to any preceding claim wherein the metal is palladium.

20. A process according to any of claims 1 to 17 wherein the racemisation catalyst comprises palladium on carbon.

21. A process according to any of claims 1 to 18 wherein the metal is ruthenium.

22. A process according to claim 21 wherein the racemisation catalyst comprises a complex of ruthenium (II) and at least one aromatic or heteroaromatic ligand.

23. A process according to Claim 22 wherein the racemisation catalyst comprises chlorodicarbonyl[1-(i-propylamino)-2,3,4,5-tetraphenylcyclopentadienyl]ruthenium. (II), 1-hydroxytetraphenylcyclopentadienyl(tetrapheyl-2,4-cyclopentadien-1-one)-mu-hydrotetracarbonyldiruthenium (II), or dichloro(p-cymene)ruthenium(II) dimer.

24. A process according to any proceeding claim wherein the second enantiomeric compound is reacted with a reagent in the presence of the enzyme to form the third compound.

25. A process according to Claim 24 wherein the reagent is an acyl donor.

26. A process according to Claim 24 or 25 wherein the reagent is a vinyl alkanoate or an isopropenyl alkanoate.

27. A process according to Claim 26 wherein the reagent is vinyl acetate.

## Patentansprüche

1. Verfahren zur stereoselektiven Herstellung einer dritten Verbindung, wobei das Verfahren umfasst:
Umsetzen eines Substrats, das eine erste enantiomere Verbindung umfasst, mit einem Racemisierungskatalysator, um eine zweite enantiomere Verbindung zu erhalten, und gleichzeitig Reagierenlassen der zweiten enantiomeren Verbindung in Gegenwart eines Enzyms, um die dritte Verbindung zu erhalten,
wobei der Racemisierungskatalysator ein Metall umfasst, wobei das Verfahren durchgeführt wird in einem Lösemittel, das wenigstens einen C₁₋₁₀ (Hydro)Fluorkohlenstoff umfasst, und wobei die erste und die zweite enantiomere Verbindung ausgewählt sind aus Alkoholen, Carbonsauren, Carbonsäureestern, Aminosäureestern, Aminen, Thiolen und Amiden.

2. Verfahren nach Anspruch 1, wobei das Enzym eine Hydrolase ist.

3. Verfahren nach Anspruch 2, wobei das Enzym ausgewählt ist aus den Proteasen und Lipasen.

4. Verfahren nach irgendeinem der Ansprüche 1 bis 3, wobei das Enzym Teil einer Kultur ganzer Zellen ist.

5. Verfahren nach irgendeinem vorausgehenden Anspruch, wobei die erste und die zweite enantiomere Verbindung Rund S-Isomere einer Verbindung sind.

6. Verfahren nach irgendeinen vorausgehenden Anspruch, wobei das Substrat ein Racemat aus der ersten und der zweiten enantiomeren Verbindung umfasst.

7. Verfahren nach irgendeinem der Ansprüche 1 bis 5, wobei das Substrat kein Racemat aus der ersten und zweiten enantiomeren Verbindung ist.

8. Verwahren nach irgendeinem vorausgehenden Anspruch, wobei der wenigstens eine C₁₋₁₀ Hydrofluorkohlenstoff aus der Gruppe ausgewählt ist, die besteht aus Difluormethan (R-32), Pentafluorethan (R-125), 1,1,1,-Trifluorethan (R-143a), 1,1,2,2-Tetrafluorethan (R-134), 1,1,1,2-Tetrafluorethan (R-134a), 1,1-Dlfluorethan (R-152a), 1,1,1,3,3-Pentafluorpropan (R-245fa), 1,1,1,2,3,3-Hexafluorpropan (R-236ea) und 1,1,1,2,3,3,3-Heptafluorpropan (R-227ea), 2,3,3,3-Tetrafluorpropen (R-1234yf), 1,3,3,3-Tetrafluorpropen (R-1234ze) und (Z)-1,2,3,3,3-Pentafluorpropen (R-1225ye).

9. Verfahren nach Anspruch 8, wobei das Lösemittel wenigstens eines von 1,1,3,2,3,3,3-Heptafluorpropan (R-227ea) und 1,1,1,2-Tetrafluorethan (R-134a) umfasst.

10. Verfahren nach irgendeinem der Ansprüche 1 bis 7, wobei das Lösemittel Iodtrifluormethan (CF₃I) umfasst.

11. Verfahren nach irgendeinem vorausgehenden Anspruch, wobei der wenigstens eine (Hydro)Fluorkohlenstoff in Kombination mit einem Co-Lösemittel verwendet wird.

12. Verfahren nach Anspruch 11, wobei das Co-Lösemittel halogenfrei ist.

13. Verfahren nach irgendeinem vorausgehenden Anspruch, wobei das Lösemittel im flüssigen Zustand vorliegt.

14. Verfahren nach irgendeinem vorausgehenden Anspruch, das in Gegenwart von Wasser in einem Anteil durchgeführt wird, der geringer ist als derjenige, der benötigt wird, damit das Wasser in dem Reaktionssystem eine separate wässrige Phase bildet.

15. Verfahren nach Anspruch 14, wobei die verwendete Menge an Wasser unterhalb des Sättigungsgrads für das Lösemittel liegt.

16. Verfahren nach Anspruch 14 oder 15, wobei die Menge an Wasser, die verwendet wird, weniger als 1 Gew.-% Wasser, bezogen auf das Gesamtgewicht des Lösemittels, beträgt.

17. Verfahren nach irgendeinem vorausgehenden Anspruch, wobei das Enzym *Subtilisin Carlsberg* ist.

18. Verfahren nach irgendeinem vorausgehenden Anspruch, wobei der Racemisierungskatalysator einen Metallkomplex umfasst.

19. Verfahren nach irgendeinem vorausgehenden Anspruch, wobei das Metall Palladium ist.

20. Verfahren nach irgendeinem der Ansprüche 1 bis 17, wobei der Racemisierungskatalysater Palladium auf Kohlenstoff umfasst.

21. Verfahren nach irgendeinem der Ansprüche 1 bis 18, wobei das Metall Ruthenium ist.

22. Verfahren nach Anspruch 21, wobei der Racemisierungskatalysator ein Komplex aus Ruthenium (II) und wenigstens einem aromatischen oder heternaromatischen Liganden umfasst.

23. Verfahren nach Anspruch 22, wobei der Racemisierungskatalysator Chlordicarbonyl[1-(i-propylamino)-2,3,4,5-tetraphenylcyclopentadienyl]ruthenium (II), 1-Hydroxytetraphenylclopentadienyl(tetraphenyl-2,4-cyclopentadien-1-on)-mu-hydrotetracarbonyldiruthenium (II) oder Dichlor(p-cymel)ruthenium(II)dimer umfasst.

24. Verfahren nach irgendeinem vorausgehenden Anspruch, wobei die zweite enantiomere Verbindung mit einem Reagens in Gegenwart des Enzyms umgesetzt wird, um die dritte Verbindung zu bilden.

25. Verfahren nach Anspruch 24, wobei das Reagens ein Acyldonor ist.

26. Verfahren nach Anspruch 24 oder 25, wobei das Reagens ein Vinylalkanoat oder ein Isopropenylalkanoat ist.

27. Verfahren nach Anspruch 26, wobei das Reagens Vinylacetat ist.

## Revendications

1. Procédé de préparation d'un troisième composé stéréosélectif, lequel procédé comprend :
la réaction d'un substrat comprenant un premier composé énantiomère avec un catalyseur de racémisation pour donner un deuxième composé énantiomère, et parallèlement, la réaction du deuxième composé énantiomère en présence d'une enzyme pour donner le troisième composé,
dans lequel le catalyseur de racémisation comprend un métal,
dans lequel le procédé est effectué dans un solvant comprenant au moins un (hydro)fluorocarbure en C₁₋₁₀, et
dans lequel les premier et deuxième composés énantiomères sont choisis parmi les alcools, les acides carboxyliques, les esters d'acide carboxylique, les esters d'acide aminé, les amines, les thiols et les amides.

2. Procédé selon la Revendication 1, dans lequel l'enzyme est une hydrolase.

3. Procédé selon la Revendication 2, dans lequel l'enzyme est choisie parmi les protéases et les lipases.

4. Procédé selon l'une quelconque des Revendications 1 à 3, dans lequel l'enzyme fait partie d'une culture cellulaire totale.

5. Procédé selon l'une quelconque des revendications précédentes, dans lequel les premier et deuxième composés énantiomères sont des isomères R et S d'un composé.

6. Procédé selon l'une quelconque des revendications précédentes, dans lequel le substrat comprend un racémate des premier et deuxième composés énantiomères.

7. Procédé selon l'une quelconque des Revendications 1 à 5, dans lequel le substrat n'est pas un racémate des premier et deuxième composés énantiomères.

8. Procédé selon l'une quelconque des revendications précédentes, dans lequel au moins un hydrofluorocarbure en C₁₋₁₀ est choisi dans le groupe constitué par le difluorométhane (R-32), le pentafluoroéthane (R-125), le 1,1,1-trifluoroéthare (R-143a), le 1,1,2,2-tétrafluoroéthane (R-134), le 1,1,1,2-tétrafluoroéthane (R-134a), le 1,1-difluoroéthane (R-152a), le 1,1,1,3,3-pentafluoropropane (R-245fa), le 1,1,1,2,3,3-hexafluoropropane (R-236ea), le 1,1,1,2,3,3,3-heptafluoropropane (R-227ea), le 2,3,3,3-tétrafluoropropène (R-1234yf), le 1,3,3,3-tétrafluoropropène (R-1234ze) et le (Z)-1,2,3,3,3-pentafluoropropène (R-1225ye).

9. Procédé selon la Revendication 8, dans lequel le solvant comprend au moins un solvant choisi parmi le 1,1,1,2,3,3,3-heptafluoropropane (R-227ea) et le 1,1,1,2-tétrafluoroéthane (R-134a).

10. Procédé selon l'une quelconque des Revendications 1 à 7, dans lequel le solvant comprend de l'iodotrifluorométhane (CF₃I).

11. Procédé selon l'une quelconque des revendications précédentes, dans lequel au moins un (hydro)fluorocarbure est utilisé en combinaison avec un cosolvant.

12. Procédé selon la Revendication 11, dans lequel le cosolvant est exempt d'halogène.

13. Procédé selon l'une quelconque des revendications précédentes, dans lequel le solvant est à l'état liquide.

14. Procédé selon l'une quelconque des revendications précédentes, qui esL effectué en présence d'eau à un taux inférieur à celui requis pour que l'eau forme une phase aqueuse distincte dans le système réactionnel.

15. Procédé selon la Revendication 14, dans lequel la quantité d'eau utilisée est inférieure au taux de saturation pour le solvant.

16. Procédé selon la Revendication 14 ou 15, dans lequel la quantité d'eau utilisée est inférieure à 1 % en poids d'eau, sur la base du poids total du solvant.

17. Procédé selon l'une quelconque des revendications précédentes, dans lequel l'enzyme est *Subtilisin Carlsberg*.

18. Procédé selon l'une quelconque des revendications précédentes, dans lequel le catalyseur de racémisation comprend un complexe métallique.

19. Procédé selon l'une quelconque des revendications précédentes, dans lequel le métal est le palladium.

20. Procédé selon l'une quelconque des Revendications 1 à 17, dans lequel le catalyseur de racémisation comprend du palladium sur carbone.

21. Procédé selon l'une quelconque des Revendications 1 à 18, dans lequel le métal est le ruthénium.

22. Procédé selon la Revendication 21, dans lequel le catalyseur de racémisation comprend un complexe de ruthénium (II) et au moins un ligand aromatique ou hétéroaromatique.

23. Procédé selon la Revendication 22, dans lequel le catalyseur de racémisation comprend du chlorodicarbonyl[1-(i-propylamino)-2,3,4,5-tétraphénylcyclopentadiényl]ruthénium (II), du 1-hydroxytétraphéylylcyclopentadiényl(tétraphényl-2,4-cyclopentadièn-1-one-mu-hydrotétracarbonyldiruthénium (II) ou un dimère de dichloro(p-cymène)ruthénium (II).

24. Procédé selon l'une quelconque des revendications précédentes, dans lequel le deuxième composé énantiomère est mis à réagir avec un réactif en présence de l'enzyme pour former le troisième composé.

25. Procédé selon la Revendication 24, dans lequel le réactif est un donneur acyle.

26. Procédé selon la Revendication 24 ou 25, dans lequel le réactif est un alcanoate de vinyle ou un alcanoate d'isopropényle.

27. Procédé selon la Revendication 24, dans lequel le réactif est l'acétate de vinyle.
